(19) Europäisches Patentamt
European Patent Office
Office européen des brevets

(11) Veröffentlichungsnummer: **0 570 398 B1**

(12) **EUROPÄISCHE PATENTSCHRIFT**

(45) Veröffentlichungstag der Patentschrift: **19.07.95**

(51) Int. Cl.[6]: **A61K 7/075**, A61K 7/08

(21) Anmeldenummer: **92903147.4**

(22) Anmeldetag: **28.01.92**

(86) Internationale Anmeldenummer:
**PCT/EP92/00179**

(87) Internationale Veröffentlichungsnummer:
**WO 92/13512 (20.08.92 92/22)**

# (54) FLIESSFÄHIGES PERLGLANZKONZENTRAT.

(30) Priorität: **06.02.91 DE 4103551**

(43) Veröffentlichungstag der Anmeldung:
**24.11.93 Patentblatt 93/47**

(45) Bekanntmachung des Hinweises auf die Patenterteilung:
**19.07.95 Patentblatt 95/29**

(84) Benannte Vertragsstaaten:
**AT BE CH DE DK ES FR GB GR IT LI NL SE**

(56) Entgegenhaltungen:
**EP-A- 0 205 922**
**EP-A- 0 300 379**
**EP-A- 0 376 083**

(73) Patentinhaber: **Henkel Kommanditgesellschaft auf Aktien**

**D-40191 Düsseldorf (DE)**

(72) Erfinder: **KAWA, Rolf**
**Fontanestrasse 28**
**D-4019 Monheim 2 (DE)**
Erfinder: **ANSMANN, Achim**
**Kirchberg 25**
**D-4006 Erkrath (DE)**
Erfinder: **STRAUSS, Gabriele**
**Elbruchstrasse 6**
**D-4000 Düsseldorf 13 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Gegenstand der Erfindung ist ein Perlglanzkonzentrat in Form einer fließ- oder pumpfähigen, wäßrigen Dispersion mit einem Gehalt von 15 - 40 Gew.-% an perlglanzbildenden Komponenten.

Wäßrigen Zubereitungen von Tensiden und kosmetischen Präparaten kann man durch Einarbeitung von Substanzen, die nach dem Abkühlen in Form feiner, perlmuttartig aussehender Kristalle ausfallen und in den Zubereitungen dispergiert bleiben, ein perlglänzendes, ästhetisch ansprechendes Aussehen verleihen. Als perlglanzbildende Stoffe eignen sich z. B. die Mono-, Di- und gegebenenfalls Triester von Ethylenglykol, Propylenglykol und oligomeren Alkylenglykolen dieser Art oder Glycerin mit $C_{14}$-$C_{22}$-Fettsäuren, Fettsäuren sowie Monoalkanolamide von Fettsäuren.

Es ist auch bekannt, die genannten Perlglanzbildner in Wasser oder in wäßrigen Tensidlösungen stabil zu dispergieren und die auf diese Weise erhaltenen konzentrierten Perlglanzdispersionen den perlglänzend auszustattenden Zubereitungen ohne Erwärmung zuzusetzen, so daß sich das für die Einarbeitung sonst erforderliche Erwärmen und Abkühlen zur Bildung der Perlglanzkristalle erübrigt.

Perlglanzkonzentrate auf Basis der genannten Perlglanzbildner sind z. B. aus DE-A-16 69 152, aus JP-56/71021 (Chem. Abstr. 95/156360), aus DE-A-34 11 328, DE-A-35 19 081 und DE-A-38 43 572 bekannt.

Ein Problem bei der Herstellung und Anwendung von Perlglanzkonzentraten stellt die Fließ- und Pumpfähigkeit dar. Vor allem bei hohen Konzentrationen an perlglanzbildenden Komponenten und Emulgatoren ist die Fließ- und Pumpfähigkeit häufig stark eingeschränkt, oder es liegen sogar Mischungen vor, die nicht fließfähig sind und auch mit den gängigen Apparaturen nicht pumpbar sind.

In der deutschen Patentanmeldung DE 38 43 572 wurde daher vorgeschlagen, die Viskosität von Perlglanzkonzentraten durch Zugabe geringer Mengen niedermolekularer, mehrwertiger Alkohole zu senken und dadurch Fließ- und Pumpfähigkeit zu erreichen.

Diese Anmeldung offenbart ein fließfähiges Perlglanzkonzentrat, dadurch gekennzeichnet, daß es :

(A) 15-40 Gew % an perlglanzbildenden Komponenten

(B) 5-55 Gew % an nichtionogenen, ampholytischen und/oder zwitterionischen Emulgatoren und

(C) 0.1-5 Gew % an niedermolekularen, mehrwertigen Alkoholen enthält.

Die Problematik der Konservierung dieser Perlglanzkonzentrate wird jedoch nur in üblicher Weise dahingehend angesprochen, daß die Konservierung mit bekannten Konservierungsmitteln empfohlen wird.

Zum Schutz vor Bakterien und Pilzbefall werden die bekannten Perlglanzkonzentrate zusätzlich mit Konservierungsmitteln, wie z.B. organischen Säuren (Ameisensäure, Benzoesäure, Salicylsäure, Sorbinsäure), Formaldehyd, Isothiazolinonen, PHB-Estern oder 1,3-Dioxanen versehen.

Konservierungsmittel sind aber nach dermatologischen Erkenntnissen Ursache einer Reihe von Hautirritationen und -erkrankungen. Daher werden in jüngster Zeit verstärkt Körperreinigungsmittel und Kosmetika angeboten, die frei von Konservierungsmitteln sind. Um solchen Produkten mit Hilfe von Perlglanzkonzentraten das vom Verbraucher gewünschte, ästhetisch ansprechende Aussehen verleihen zu können, besteht somit ein Bedarf an Perlglanzkonzentraten, die ohne Konservierungsmittel formuliert sind. Diese Freiheit von Konservierungsmitteln sollte sich aber nicht negativ auf die mikrobiologische Stabilität und die anderen Eigenschaften dieser Konzentrate auswirken.

Es wurde nun gefunden, daß durch Zugabe von größeren Mengen niedermolekularer, mehrwertiger Alkohole konservierungsmittelfreie Perlglanzkonzentrate erhalten werden, die sowohl hinsichtlich ihrer Haltbarkeit als auch ihrer Fließ- und Pumpfähigkeit den bekannten Perlglanzkonzentraten entsprechen.

Gegenstand der Anmeldung ist somit ein Perlglanzkonzentrat in Form einer fließfähigen, wäßrigen Dispersion, das gekennzeichnet ist durch einen Gehalt von

(A) 15 - 40 Gew.-% an perlglanzbildenden Komponenten

(B) 5 - 55 Gew.-% an Emulgatoren und

(C) 15 - 40 Gew.-% an Alkoholen mit 2 - 6 C-Atomen und 2 - 6 Hydroxylgruppen.

Besonders vorteilhafte Eigenschaften zeigen Perlglanzkonzentrate mit einem Gehalt von

(A) 20 - 30 Gew.-% an perlglanzbildenden Komponenten

(B) 10 - 35 Gew.-% an Emulgatoren und

(C) 20 - 40 Gew.-% an Alkoholen mit 2 - 6 C-Atomen und 2 - 6 Hydroxylgruppen.

Unter perlglanzbildenden Komponenten werden schmelzbare Fett- oder Wachskörper verstanden, die beim Abkühlen ihrer wäßrigen Lösungen oder Emulsionen in einem Temperaturbereich von etwa 30 - 90 °C in Form feiner, perlglänzender Körper auskristallisieren.

Zu diesen schmelzbaren Fett- oder Wachskörpern gehören

(A1) Ester der Formel (I),

$$R^1 - (OC_nH_{2n})_x - OR^2 \qquad (I)$$

in der $R^1$ eine lineare Fettacylgruppe mit 14 bis 22 C-Atomen, $R^2$ Wasserstoff oder eine Gruppe $R^1$, n = 2 oder 3 und x eine Zahl von 1 bis 4 ist,
(A2) Monoalkanolamide der allgemeinen Formel (II),

$R^3$ - CO -NH - X (II)

in der $R^3$ eine Alkylgruppe mit 8 bis 22 C-Atomen, insbesondere mit 8 bis 18 C-Atomen, und X eine Gruppe $-CH_2-CH_2-OH$, eine Gruppe $-CH_2-CH_2-CH_2-OH$ oder eine Gruppe $-C(CH_3)_2-OH$ darstellt,
(A3) lineare, gesättigte Fettsäuren mit 14 bis 22 C-Atomen,
(A4) $\beta$-Ketosulfone der allgemeinen Formel (III),

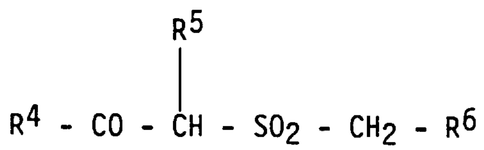
(III)

in der $R^4$ eine Alkyl- oder Alkenylgruppe mit 11 bis 21 C-Atomen, $R^5$ und $R^6$ Wasserstoffatome oder gemeinsam eine Ethylengruppe, die mit der zwischen $R^5$ und $R^6$ liegenden Gruppe einen Tetrahydrothiophendioxidring bildet, darstellen
sowie
(A5) Mono-, Di- und Triester des Glycerins mit linearen, gesättigten Fettsäuren mit 14 bis 22 C-Atomen.

Als Ester (A1) der allgemeinen Formel $R^1(OC_nH_{2n})_xOR^2$ können z. B. die Mono- und Diester des Ethylenglykols und Propylenglykols mit höheren Fettsäuren, z. B. mit Palmitinsäure, Stearinsäure oder Behensäure, oder die Diester des Diethylenglykols oder des Triethylenglykols mit solchen Fettsäuren eingesetzt werden. Geeignet sind auch Mischungen von Mono- und Diestern der genannten Glykole mit Fettsäuregemischen, z. B. mit gehärteter Talgfettsäure, Palmfettsäure oder mit der gesättigten $C_{14}$-$C_{18}$-Fettsäurefraktion der Talgfettsäure. Bevorzugt geeignet sind der Ethylenglykolmono- und/oder -diester der Palmitin- und/oder Stearinsäure.

Bevorzugte Monoalkanolamide (A2) sind die Monoethanolamide. Diese Verbindungen können einen einheitlichen Alkylrest enthalten. Es ist jedoch üblich, bei der Herstellung der Alkanolamide von Fettsäuregemischen aus natürlichen Quellen, z.B. Kokosfettsäuren, auszugehen, so daß entsprechende Mischungen bezüglich der Alkylreste vorliegen.

Als lineare Fettsäuren (A3) können z. B. Palmitinsäure, Stearinsäure, Arachinsäure oder Behensäure eingesetzt werden, geeignet sind aber auch technische Fettsäureschnitte, die ganz oder überwiegend aus Fettsäuren mit 16 bis 22 C-Atomen bestehen, z. B. Palmitin-Stearinsäure-Fraktionen, wie sie aus Talgfettsäure oder Palmfettsäure durch Abtrennung der bei +5 °C flüssigen Fettsäuren gewonnen werden oder Palmitin-Stearinsäure-Fraktionen, wie sie durch Härten von Talgfettsäure oder Palmfettsäure erhältlich sind.

Gegenüber den bekannten Ethylenglykolmono- und -diestern haben die $\beta$-Ketosulfone (A4) der allgemeinen Formel (III) den Vorteil, daß der Perlglanz der Zubereitungen eine höhere Thermostabilität aufweist, d.h. daß der Perlglanz beim Erwärmen der Zubereitungen auf über 50 °C, teilweise sogar auf über 70 °C, über mehrere Stunden erhalten bleibt. Hinsichtlich weiterer Informationen zu den genannten $\beta$-Ketosulfonen wird ausdrücklich auf den Inhalt der deutschen Patentanmeldung 35 08 051 hingewiesen.

Zu den in der erfindungsgemäßen Lehre verwendbaren Estern des Glycerins (A5) gehören die Mono-, Di- und insbesondere Triester mit Myristinsäure, Palmitinsäure, Stearinsäure und Behensäure, sowie mit Mischungen dieser Fettsäuren.

Ebenfalls als perlglanzbildende Komponenten eingesetzt werden können Fettalkohole mit mindestens 20 C-Atomen, insbesondere mit 20 bis 30 C-Atomen.

Die erfindungsgemäßen Perlglanzkonzentrate können sowohl ausschließlich Vertreter einer dieser Verbindungsklassen als auch Mischungen von Vertretern mehrerer dieser Verbindungsklassen enthalten.

Bevorzugte perlglanzbildende Komponenten sind Vertreter der Klassen (A1) bis (A3).

Fettsäure-mono- oder -dialkanolamide, also perlglanzbildende Komponenten der Gruppe (A2), und deren Derivate werden aber in jüngster Zeit verdächtigt, an der Bildung von Nitroaminen beteiligt zu sein. Es kann daher erwünscht sein, kosmetische Zubereitungen ohne solche Alkanolamine und Alkanolamin-Derivate zu formulieren. Aus diesem Grunde können Verbindungen der Klassen (A1) und (A3) besonders bevorzugte perlglanzbildende Komponenten sein.

Ganz besonders bevorzugt sind solche Perlglanzkonzentrate, deren perlglanzbildende Komponenten zu mindestens 70 Gew.-%, insbesondere zu mindestens 90 Gew.-%, aus Ethylenglykoldistearat bestehen.

Als Emulgatoren (B) können ionogene oder nichtionogene grenzflächenaktive Verbindungen verwendet werden, die sich durch eine lipophile, bevorzugt lineare, Alkyl- oder Alkenylgruppe und mindestens eine hydrophile Gruppe auszeichnen. Diese hydrophile Gruppe kann sowohl eine ionogene als auch eine nichtionogene Gruppe sein.

Erfindungsgemäß einzusetzende anionische Emulgatoren (B) sind beispielsweise Alkylsulfate und Alkylpolyethylenglykolethersulfate mit 8 bis 22 C-Atomen in der Alkylkette und 1 bis 15, insbesondere 1 bis 6 Ethylenglykolethergruppen im Molekül, die in Form ihrer Alkali-, Magnesium-, Ammonium-, Mono-, Di- oder Trialkanolammoniumsalze mit 2 bis 3 C-Atomen in der Alkanolgruppe eingesetzt werden. Weitere geeignete Aniontenside sind Alkansulfonate, $\alpha$-Olefinsulfonate, $\alpha$-Sulfofettsäuremethylester, Fettalkohol-(polyglykolether)carboxylate, Sulfobernsteinsäuremono- und -dialkylester, Sulfobernsteinsäureester-Salze, Acylisethionate, Acyltauride und Acylsarcoside, jeweils mit 8 bis 22, insbesondere 12 bis 18, C-Atomen in der Alkyl- bzw. Acylkette. Auch Seifen können als Emulgatoren verwendet werden. Dies kann z. B. dadurch erreicht werden, daß man einen kleinen Teil, d.h. etwa 1 bis 20 Gew.-%, der linearen, gesättigten Fettsäuren durch zugesetztes Alkalihydroxid verseift und auf diese Weise in einen anionischen Emulgator überführt.

Bevorzugte anionische Emulgatoren sind die Alkylpolyethylenglykolethersulfate wie beispielsweise das Natriumlaurylpolyglykolethersulfat.

Als kationische Emulgatoren (B) sind quartäre Ammoniumtenside, beispielsweise Alkyltrimethylammoniumchloride und Dialkyldimethylammoniumchloride, z. B. Cetyltrimethylammoniumchlorid, Stearyltrimethylammoniumchlorid, Distearyldimethylammoniumchlorid, Lauryldimethylammoniumchlorid und Lauryldimethylbenzylammoniumchlorid, Cetylpyridiniumchlorid und Talgalkyltris(oligooxyalkyl)-ammonium-phosphat zu nennen.

Weiterhin können als Emulgatoren (B) zwitterionische Tenside verwendet werden. Als zwitterionische Tenside werden solche oberflächenaktiven Verbindungen bezeichnet, die im Molekül mindestens eine quartäre Ammoniumgruppe und mindestens eine -$COO^{(-)}$- oder -$SO_3^{(-)}$-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammonium-glycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Ebenfalls geeignete Emulgatoren (B) sind ampholytische Tenside. Unter ampholytischen Tensiden werden solche oberflächenaktiven Verbindungen verstanden, die außer einer $C_8$-$C_{18}$-Alkyl- oder -Acylgruppe im Molekül mindestens eine freie Aminogruppe und mindestens eine -COOH- oder -$SO_3H$-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das $C_{12-18}$-Acylsarcosin.

Schließlich können auch nichtionogene Emulgatoren (B) verwendet werden, die als hydrophile Gruppe z. B. eine Polyolgruppe, eine Polyalkylenglykolethergruppe oder eine Kombination aus Polyol- und Polyglykolethergruppe enthalten. Solche Verbindungen sind beispielsweise

(B1) Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,

(B2) $C_{12}$-$C_{22}$-Fettsäuremono- und -diester von Anlagerungsprodukten von 1 bis 30 Mol Ethylenoxid an Glycerin,

(B3) Glycerinmono- und -diester und Sorbitanmono- und -diester von gesättigten und ungesättigten $C_8$-$C_{22}$-Fettsäuren und deren Ethylenoxidanlagerungsprodukte,

(B4) $C_8$-$C_{22}$-Alkylmono- und -oligoglycoside und deren ethoxylierte Analoga

und

(B5) Anlagerungsprodukte von 5 bis 60 Mol Ethylenoxid an Ricinusöl und gehärtetes Ricinusöl.

Ebenfalls geeignet sind Mischungen von Verbindungen aus mehreren dieser Substanzklassen.

Die Anlagerungsprodukte von Ethylenoxid und/oder von Propylenoxid an Fettalkohole, Fettsäuren, Alkylphenole, Glycerin-mono- und -diester sowie Sorbitanmono- und -diester von Fettsäuren oder an

Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht.

$C_{12}$-$C_{22}$-Fettsäuremono- und -diester von Anlagerungsprodukten von Ethylenoxid an Glycerin sind aus DE-PS 20 24 051 als Rückfettungsmittel für kosmetische Zubereitungen bekannt. $C_8$-$C_{22}$-Alkylmono- und -oligoglycoside, ihre Herstellung und ihre Verwendung als oberflächenaktive Stoffe sind beispielsweise aus US-A 3,839,318, US-A 3,707,535, US-A 3,547,828, DE-A-19 43 689, DE-A 20 36 472 und DE-A 30 01 064 sowie EP-A 77 167 bekannt. Ihre Herstellung erfolgt insbesondere durch Umsetzung von Glucose oder Oligosacchariden mit primären Alkoholen mit 8 bis 22 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Oligomerisierungsgrade von 1,4 und kleiner können besonders bevorzugt sein. Der Oligomerisierungsgrad ist dabei ein statistischer Mittelwert, dem eine für solche technischen Produkte übliche Homologenverteilung zugrunde liegt.

Die Verbindungen aus der Gruppe (B1) stellen besonders bevorzugte nichtionogene Emulgatoren (B) im Rahmen der erfindungsgemäßen Lehre dar.

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen und tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Im Rahmen der erfindungsgemäßen Lehre können die Perlglanzkonzentrate Vertreter einer oder mehrerer der genannten Tensidklassen enthalten.

Da Perlglanzkonzentrate überwiegend zu Formulierungen mit anionischen Tensiden hinzugegeben werden, sind anionische Emulgatoren bevorzugte Emulgatoren (B).

Weiterhin haben sich die erfindungsgemäßen Perlglanzkonzentrate, die lediglich nichtionogene, zwitterionische und/oder ampholytische Tenside enthalten, als besonders universell einsetzbar und mit wäßrigen Zubereitungen wasserlöslicher oberflächenaktiver Stoffe beliebiger Art und Ionogenität als besonders gut verträglich erwiesen. Die Verwendung dieser Tensidklassen ist daher ebenfalls bevorzugt.

Entscheidend für die Fließ- bzw. Pumpfähigkeit der erfindungsgemäßen Perlglanzkonzentrate sowie ihre Beständigkeit gegenüber Bakterien und Pilzbefall ist der Gehalt an Alkoholen mit 2-6 Kohlenstoffatomen und 2-6 Hydroxylgruppen. Solche Alkohole sind beispielsweise Ethylenglykol, 1,2- und 1,3-Propylenglykol, Glycerin, Di- und Tri-ethylenglykol, Erythrit, Arabit, Adonit, Xylit, Sorbit, Mannit und Dulcit. Die Verwendung von Glycerin, 1,2-Propylenglykol, 1,3-Propylenglykol und/oder Sorbit ist besonders bevorzugt.

Die Verwendung von Glycerin als Alkohol führt zu Perlglanzkonzentraten, die den Endprodukten einen besonders brillanten Perlglanz verleihen.

Daneben enthalten die erfindungsgemäßen Perlglanzkonzentrate im wesentlichen Wasser.

Weiterhin können in untergeordneten Mengen Puffersubstanzen zur Einstellung des pH-Wertes auf Werte zwischen 2 und 8, z. B. Citronensäure und/oder Natriumcitrat, sowie anorganische Salze, beispielsweise Natriumchlorid, als Verdickungsmittel enthalten sein.

Wenngleich die Perlglanzkonzentrate üblicherweise konservierungsmittelfrei sind, so können doch in Ausnahmefällen untergeordnete Mengen handelsüblicher Konservierungsmittel, die beispielsweise über einzelne Rohstoffe eingebracht wurden, enthalten sein.

Die erfindungsgemäßen Perlglanzkonzentrate sind mindestens in einem Temperaturbereich von 5 - 40 °C pumpbar und über einen längeren Zeitraum , d. h. mindestens etwa 3 Monate, lagerstabil.

Die Herstellung der erfindungsgemäßen Perlglanzkonzentrate kann so erfolgen, daß die Komponenten (A), (B) und (C) zunächst gemeinsam auf eine Temperatur erwärmt werden, die etwa 1 bis 30 °C oberhalb des Schmelzpunktes liegt. Dies wird in den meisten Fällen eine Temperatur von etwa 60 bis 90 °C sein. Sodann wird zu dieser Mischung das auf etwa die gleiche Temperatur erwärmte Wasser hinzugegeben. Falls als Emulgator ein ionisches, wasserlösliches Tensid eingesetzt wird, kann es bevorzugt sein, dieses in

der Wasserphase aufzulösen und mit dem Wasser zusammen in die Mischung einzubringen. Die wäßrige Phase kann auch bereits gegebenenfalls die Puffersubstanzen gelöst enthalten. Die entstehende Dispersion wird dann unter stetigem Rühren auf Raumtemperatur, d. h. auf etwa 25 °C, abgekühlt. Die Viskosität des Perlglanzkonzentrates ist in den allermeisten Fällen so niedrig, daß auf den Einsatz besonderer Rühraggregate wie Homogenisatoren oder andere hochtourige Mischvorrichtungen verzichtet werden kann.

Die erfindungsgemäßen Perlglanzkonzentrate eignen sich zur Herstellung getrübter und perlglänzender flüssiger, wäßriger Zubereitungen wasserlöslicher oberflächenaktiver Stoffe. Sie können z.B. in flüssige Wasch- und Reinigungsmittel wie Spülmittel, flüssige Feinwaschmittel und flüssige Seifen, bevorzugt aber in flüssige Körperreinigungs- und Pflegemittel wie z.B. Haarwaschmittel (Shampoos), flüssige Hand- und Körperwaschmittel, Duschbadzubereitungen, Badezusätze (Schaumbäder), Haarspülmittel oder Haarfärbezubereitungen eingearbeitet werden.

Zur Erzeugung von Perlglanz werden den klaren wäßrigen Zubereitungen bei 0 bis 40 °C die erfindungsgemäßen Perlglanzkonzentrate in einer Menge von 0,5 bis 10 Gew.-%, insbesondere 1,5 bis 5 Gew.-%, der Zubereitung zugesetzt und unter Rühren darin verteilt. Je nach Zubereitung und Einsatzkonzentration entsteht ein metallisch glänzender, dichter bis schwach glänzender, extrem dichter Perlglanz.

Die folgenden Beispiele sollen den Erfindungsgegenstand näher erläutern, ohne ihn darauf zu begrenzen.

## Beispiele

### 1. Perlglanzkonzentrate

Es wurden Perlglanzkonzentrate mit den in Tabelle 1 aufgeführten Zusammensetzungen hergestellt. Bei den mit den Verkaufsbezeichnungen aufgeführten Komponenten handelt es sich um die im folgenden aufgeführten Substanzen.

[1] Ethylenglykoldistearat (mind. 90 % Diester) (HENKEL)

[2] Kokosfettsäuremonoethanolamid (CTFA-Bezeichnung: Cocamide MEA (ca. 95 % Amid)) (HENKEL)

| | |
|---|---|
| Zusammensetzung der Fettsäure: | ca. 56 % Laurinsäure<br>ca. 21 % Myristinsäure<br>ca. 10 % Palmitinsäure<br>ca. 13 % Stearinsäure und Ölsäure |

[3] Palmitinsäure/Stearinsäure-Mischung (ca. 1:1)

[4] Fettalkoholpolyglycolether (CTFA-Bezeichnung: Laureth-10 (ca. 74 % C12/C14-Fettalkohol)) (HENKEL)

[5] Natriumlaurylethersulfat (CTFA-Bezeichnung: Sodium Laureth Sulfate (ca. 72 % Aktivsubstanz)) (HENKEL).

[6] $C_{12}$-$C_{14}$-Fettalkohol + 4 Ethylenoxid (HENKEL)

[7] Wäßrige Lösung eines Fettsäureamid-Derivats mit Betainstruktur der Formel $R\text{-}CONH\text{-}(CH_2)_3\text{-}N^+(CH_3)_2\text{-}CH_2\text{-}COO^-$ (CTFA-Bezeichnung: Cocamidopropyl Betaine (ca. 30 % Aktivsubstanz, ca. 5 % NaCl)) (HENKEL)

[8] $C_{12}$-$C_{14}$-Fettalkohol + 3 Ethylenoxid (HENKEL)

[9] Polyol-Fettsäureester (CTFA-Bezeichnung: PEG-7-Glyceryl Cocoate) (Henkel)

[10] 86 % in Wasser

## Tabelle 1: Fließfähige Perlglanzkonzentrate

Gehalt [Gew.-%]

| Komponenten | Mischung Nr. 1 | 2 | 3 | 4 | 5 | 6 | 7 | 8 | 9 | 10 |
|---|---|---|---|---|---|---|---|---|---|---|
| Perlglanzbildner (A) | 23 | 30 | 25 | 25 | 25 | 23 | 25 | 23 | 20 | 30 |
| davon: | | | | | | | | | | |
| Cutina(R) AGS[1] | 20 | 25 | 25 | 25 | 25 | 20 | 25 | 20 | - | 20 |
| Comperlan(R) 100[2] | 3 | 5 | - | - | - | 3 | - | 3 | - | - |
| Fettsäure 16/18[3] | - | - | - | - | - | - | - | - | 20 | 10 |
| Emulgator (B) | 22 | 26 | 17 | 17 | 19 | 22 | 18,5 | 22 | 10 | 35 |
| davon: | | | | | | | | | | |
| Dehydol(R) 100[4] | 2 | 5 | - | - | - | 2 | - | 2 | 5 | - |
| Texapon(R) N70[5] | 20 | 21 | - | - | - | 20 | - | 20 | - | 30 |
| Dehydol(R) LS4[6] | - | - | 11 | 11 | 5 | - | 11 | - | - | 5 |
| Dehyton(R) K[7] | - | - | 6 | 6 | 9 | - | 7,5 | - | - | - |
| Dehydol(R) LS3[8] | - | - | - | - | 5 | - | - | - | - | - |
| Cetiol(R) HE[9] | - | - | - | - | - | - | - | - | 5 | - |
| Alkohol (C) | 20 | 20 | 30 | 30 | 20 | 30 | 20 | 30 | 40 | 30 |
| davon: | | | | | | | | | | |
| Glycerin[10] | 20 | - | 10 | 30 | - | - | 20 | - | 20 | 30 |
| 1,2-Propylenglykol | - | 20 | 10 | - | - | - | - | 20 | - | - |
| 1,3-Propylenglykol | - | - | - | - | 20 | - | - | - | 20 | - |
| Sorbit | - | - | 10 | - | - | 30 | - | 10 | - | - |
| Wasser Stellmittel | <—— | | | | a d | 1 0 0 | | | | ——> |

## 2. Anwendungsbeispiele

1) Duschbad

| | Gewichtsteile |
|---|---|
| Texapon$^R$N 25[11] | 35 |
| Dehyton$^R$K | 7 |
| Lamepon$^R$S[12] | 5 |
| Perlglanzkonzentrat 1 | 3 |
| Wasser | ad 100 |

Anschließend wurde die Viskosität des Duschbades durch Zugabe von Natriumchlorid auf einen Wert von 4000 mPas eingestellt.

[11] Natriumlaurylethersulfat (CTFA-Bezeichnung: Sodium Laureth Sulfate (ca. 28% Aktivsubstanz in Wasser)) (HENKEL)

[12] Eiweisshydrolysat-Fettsäure-Kondensat-Kalium-Salz (CTFA-Bezeichnung: Potassium Coco-Hydrolyzed Animal Collagen)
(HENKEL)

2) mildes Shampoo

| | Gewichtsteile |
|---|---|
| Texapon$^R$ASV[13] | 14 |
| Texapon$^R$SB 3[14] | 10 |
| Dehyton$^R$G[15] | 14 |
| Nutrilan$^R$I 50[16] | 2,5 |
| Perlglanzkonzentrat 2 | 2 |
| Wasser | ad 100 |

Anschließend wurde die Viskosität durch Zugabe von Natriumchlorid und Arlypon$^R$F (ethoxylierter Fettalkohol mit eingeengter Homologenverteilung) (HENKEL)) auf einen Wert von 5000 mPas eingestellt.

[13] Gemisch spezieller Fettalkoholethersulfate (CTFA-Bezeichnung: Sodium Laureth Sulfate (and) Magnesium Laureth Sulfate (and) Sodium Laureth 8-Sulfate (and) Magnesium Laureth 8-Sulfate (and) Sodium Oleth Sulfate (and) Magnesium Oleth Sulfate (ca. 30 % Aktivsubstanz in Wasser)) (HENKEL)

[14] Sulfobernsteinsäurehalbester auf Basis eines Alkylpolyglycolethers, Di-Na-Salz (CTFA-Bezeichnung: Disodium Laurethsolfosuccinate (ca. 40 % Aktivsubstanz in Wasser)) (HENKEL)

[15] Fettsäureamidderivat mit amphoterem Charakter (N-Hydroxyethyl-N-Kokoalkylamidoethyl-glycinat, Na-Salz) (CTFA-Bezeichnung: Cocoamphodiacetate (ca. 30 % Aktivsubstanz in Wasser)) (HENKEL)

[16] partielle Eiweisshydrolysat aus abgebautem Rinderkollagen (CTFA-Bezeichnung: Hydrolyzed Animal Protein) (HENKEL)

3) Schaumbad

| | Gewichtsteile |
|---|---|
| Texapon$^R$N 40[17] | 40 |
| Dehyton$^R$K | 6,5 |
| Dehyton$^R$G | 6,5 |
| Cetiol$^R$HE | 5 |
| Perlglanzkonzentrat 3 | 5 |
| Wasser | ad 100 |

Anschließend wurde die Viskosität durch Zugabe von Natriumchlorid auf 6000 mPas eingestellt.

[17] Natriumlaurylethersulfat (CTFA-Bezeichnung: Sodium Laureth Sulfate (ca. 28 % Aktivsubstanz in Wasser)) (HENKEL)

## Patentansprüche

1. Perlglanzkonzentrat in Form einer fließfähigen, wäßrigen Dispersion, dadurch gekennzeichnet, daß es
   (A) 15 - 40 Gew.-% an perlglanzbildenden Komponenten
   (B) 5 - 55 Gew. -% an Emulgatoren und
   (C) 15 - 40 Gew.-% an Alkoholen mit 2 - 6 C-Atomen und 2 - 6 Hydroxylgruppen
   enthält.

2. Perlglanzkonzentrat nach Anspruch 1, dadurch gekennzeichnet, daß es
   (A) 20 - 30 Gew.-% an perlglanzbildenden Komponenten
   (B) 10 - 35 Gew.-% an Emulgatoren und
   (C) 20 - 40 Gew.-% an Alkoholen mit 2 - 6 C-Atomen und 2 - 6 Hydroxylgruppen
   enthält.

3. Perlglanzkonzentrat nach einem der Ansprüche 1 oder 2, dadurch gekennzeichnet, daß als perlglanzbildende Komponenten
   (A1) Ester der Formel (I),

   $R^1 - (OC_nH_{2n})_x - OR^2$ (I)

   in der $R^1$ eine lineare Fettacylgruppe mit 14 bis 22 C-Atomen, $R^2$ Wasserstoff oder eine Gruppe $R^1$, n = 2 oder 3 und x eine Zahl von 1 bis 4 ist,
   und/oder
   (A2) Monoalkanolamide der allgemeinen Formel (II),

   $R^3$ - CO -NH - X (II)

   in der $R^3$ eine Alkylgruppe mit 8 bis 22 C-Atomen, insbesondere mit 8 bis 18 C-Atomen, und X eine Gruppe $-CH_2-CH_2-OH$, eine Gruppe $-CH_2-CH_2-CH_2-OH$ oder eine Gruppe $-C(CH_3)_2-OH$ darstellt,
   und/oder
   (A3) lineare, gesättigte Fettsäuren mit 14 bis 22 C-Atomen
   enthalten sind.

4. Perlglanzkonzentrat nach Anspruch 3, dadurch gekennzeichnet, daß die perlglanzbildenden Komponenten zu mindestens 70 Gew.-%, insbesondere zu mindestens 90 Gew.-%, aus Ethylenglykoldistearat bestehen.

5. Perlglanzkonzentrat nach einem der Ansprüche 1 bis 4, dadurch gekennzeichnet, daß als Emulgatoren nichtionogene, ampholytische und/oder zwitterionische Emulgatoren enthalten sind.

6. Perlglanzkonzentrat nach einem der Ansprüche 1 bis 5, dadurch gekennzeichnet, daß als Emulgatoren nichtionogene Tenside, insbesondere aus der Gruppe der
   (B1) Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
   (B2) $C_{12}$-$C_{22}$-Fettsäuremono- und -diester von Anlagerungsprodukten von 1 bis 30 Mol Ethylenoxid an Glycerin,
   (B3) Glycerinmono- und -diester und Sorbitanmono- und -diester von gesättigten und ungesättigten $C_8$-$C_{22}$-Fettsäuren und deren Ethylenoxidanlagerungsprodukte,
   (B4) $C_8$-$C_{22}$-Alkylmono- und -oligoglycoside und deren ethoxylierte Analoga
   und
   (B5) Anlagerungsprodukte von 5 bis 60 Mol Ethylenoxid an Ricinusöl und gehärtetes Ricinusöl,
   enthalten sind.

7. Perlglanzkonzentrat nach einem der Ansprüche 1 bis 5, dadurch gekennzeichnet, daß als Emulgatoren zwitterionische Tenside, insbesondere ausgewählt aus der Gruppe der Betaine, enthalten sind.

8. Perlglanzkonzentrat nach einem der Ansprüche 1 bis 4, dadurch gekennzeichnet, daß als Emulgatoren anionische Emulgatoren enthalten sind.

9. Perlglanzkonzentrat nach einem der Ansprüche 1 bis 8, dadurch gekennzeichnet, daß der Alkohol ausgewählt ist aus der Gruppe, die aus den Substanzen Glycerin, 1,2-Propylenglykol, 1,3-Propylenglykol und Sorbit besteht.

10. Perlglanzkonzentrat nach einem der Ansprüche 1 bis 9, dadurch gekennzeichnet, daß als Alkohol Glycerin enthalten ist.

11. Verfahren zur Herstellung von Perlglanzkonzentraten nach einem der Ansprüche 1 bis 10, dadurch gekennzeichnet, daß man eine Mischung der Komponenten (A), (B) und (C) auf eine Temperatur erwärmt, die 1 bis 30 °C oberhalb des Schmelzpunktes der Mischung liegt, mit der notwendigen Menge Wasser etwa der gleichen Temperatur mischt und anschließend auf Raumtemperatur abkühlt.

12. Verfahren zur Herstellung getrübter und perlglänzender flüssiger, wäßriger Zubereitungen wasserlöslicher oberflächenaktiver Stoffe, dadurch gekennzeichnet, daß man den klaren wäßrigen Zubereitungen bei 0 bis 40 °C Perlglanzkonzentrate nach einem der Ansprüche 1 bis 10 in einer Menge von 0,5 bis 10 Gew.-%, insbesondere von 1,5 bis 5 Gew.-%, der Zubereitung zusetzt und unter Rühren darin verteilt.

## Claims

1. A pearlescent concentrate in the form of a free-flowing, aqueous dispersion, characterized in that it contains
   (A) 15 to 40% by weight of pearlescing components,
   (B) 5 to 55% by weight of emulsifiers and
   (C) 15 to 40% by weight of alcohols containing 2 to 6 carbon atoms and 2 to 6 hydroxyl groups.

2. A pearlescent concentrate as claimed in claim 1, characterized in that it contains
   (A) 20 to 30% by weight of pearlescing components,
   (B) 10 to 35% by weight of emulsifiers and
   (C) 20 to 40% by weight of alcohols containing 2 to 6 carbon atoms and 2 to 6 hydroxyl groups.

3. A pearlescent concentrate as claimed in claim 1 or 2, characterized in that it contains as pearlescing components
   (A1) esters corresponding to formula (I):

   $R^1 - (OC_nH_{2n})_x - OR^2$ (I)

   in which $R^1$ is a linear $C_{14-22}$ fatty acyl group, $R^2$ is hydrogen or a group $R^1$, n = 2 or 3 and x is a number of from 1 to 4,
   and/or
   (A2) monoalkanolamides corresponding to general formula (II):

   $R^3 - CO - NH - X$ (II)

   in which $R^3$ is an alkyl group containing 8 to 22 and, more especially, 8 to 18 carbon atoms and X is a $-CH_2-CH_2-OH$ group, a $-CH_2-CH_2-CH_2-OH$ group or a $-C(CH_3)_2-OH$ group,
   and/or
   (A3) linear, saturated $C_{14-22}$ fatty acids.

4. A pearlescent concentrate as claimed in claim 3, characterized in that at least 70% by weight and, in particular, at least 90% by weight of the pearlescing components consist of ethylene glycol distearate.

5. A pearlescent concentrate as claimed in any of claims 1 to 4, characterized in that it contains nonionic, ampholytic and/or zwitterionic emulsifiers as emulsifiers.

**6.** A pearlescent concentrate as claimed in any of claims 1 to 5, characterized in that the emulsifiers present are nonionic surfactants, more particularly from the group consisting of

(B1) adducts of 2 to 30 moles of ethylene oxide and/or 0 to 5 moles of propylene oxide with linear $C_{8-22}$ fatty alcohols, with $c_{12-22}$ fatty acids and with alkyl phenols containing 8 to 15 carbon atoms in the alkyl group,

(B2) $C_{12-22}$ fatty acid monoesters and diesters of adducts of 1 to 30 moles of ethylene oxide with glycerol,

(B3) glycerol monoesters and diesters and sorbitan monoesters and diesters of saturated and unsaturated $C_{8-22}$ fatty acids and ethylene oxide adducts thereof,

(B4) $C_{8-22}$ alkyl mono- and -oligoglycosides and ethoxylated analogs thereof and

(B5) adducts of 5 to 60 moles of ethylene oxide with castor oil and hydrogenated castor oil.

**7.** A pearlescent concentrate as claimed in any of claims 1 to 5, characterized in that zwitterionic surfactants, more especially selected from the group of betaines, are present as emulsifiers.

**8.** A pearlescent concentrate as claimed in any of claims 1 to 4, characterized in that anionic emulsifiers are present as emulsifiers.

**9.** A pearlescent concentrate as claimed in any of claims 1 to 8, characterized in that the alcohol is selected from the group consisting of glycerol, 1,2-propylene glycol, 1,3-propylene glycol and sorbitol.

**10.** A pearlescent concentrate as claimed in any of claims 1 to 9, characterized in that glycerol is present as the alcohol.

**11.** A process for the production of the pearlescent concentrates claimed in any of claims 1 to 10, characterized in that a mixture of components (A), (B) and (C) is heated to a temperature 1 to 30 °C above the melting point of the mixture, mixed with the necessary quantity of water at substantially the same temperature and the resulting mixture is subsequently cooled to room temperature.

**12.** A process for the production of clouded and pearlescent free-flowing, aqueous preparations of water-soluble surfactants, characterized in that the pearlescent concentrates claimed in any of claims 1 to 10 are added to the clear aqueous preparations at 0 to 40 °C in a quantity of from 0.5 to 10% by weight and more especially in a quantity of from 1.5 to 5% by weight and are dispersed therein with stirring.

## Revendications

**1.** Concentré à éclat nacré sous forme d'une dispersion aqueuse fluide, caractérisé en ce qu'il contient:

(A) de 15 à 40% en poids de composants assurant un éclat nacré;

(B) de 5 à 55% en poids d'émulsifiants; et

(C) de 15 à 40% en poids d'alcools ayant de 2 à 6 atomes de carbone et de 2 à 6 groupes hydroxyle.

**2.** Concentré à éclat nacré selon la revendication 1, caractérisé en ce qu'il contient:

(A) de 20 à 30% en poids de composants assurant un éclat nacré;

(B) de 10 à 35% en poids d'émulsifiants; et

(C) de 20 à 40% en poids d'alcools avec de 2 à 6 atomes de carbone et de 2 à 6 groupes hydroxyle.

**3.** Concentré à éclat nacré selon l'une des revendications 1 ou 2, caractérisé en ce qu'il contient, comme composant assurant un éclat nacré:

(A1) Des esters de formule (I)

$$R^1\text{-}(OC_nH_{2n})_x\text{-}OR^2 \qquad (I)$$

dans laquelle $R^1$ représente un groupe acyle gras linéaire avec de 14 à 22 atomes de carbone, $R^2$ de l'hydrogène ou un groupe $R^1$, n = 2 ou 3 et x représente un nombre compris entre 1 et 4; et/ou

(A2) Des monoalcanolamides de formule générale (II)

$R^3$-CO-NH-X (II)

dans laquelle $R^3$ représente un groupe alkyle avec de 8 à 22 atomes de carbone, et en particulier avec de 8 à 18 atomes de carbone et X représente un groupe $-CH_2-CH_2-OH$, un groupe $-CH_2-CH_2-CH_2-OH$ ou un groupe $-C(CH_3)_2-OH$;
et/ou
(A3) Des acides gras linéaires saturés avec de 14 à 22 atomes de carbone.

**4.** Concentré à éclat nacré selon la revendication 3, caractérisé en ce que les composants assurant un éclat nacré sont constitués d'au moins 70% en poids et, en particulier, d'au moins 90% en poids de distéarate d'éthylèneglycol.

**5.** Concentré à éclat nacré selon l'une des revendications 1 à 4, caractérisé en ce que les émulsifiants contenus sont des émulsifiants non ionogènes, ampholytiques et/ou zwitterioniques.

**6.** Concentré à éclat nacré, selon l'une des revendications 1 à 5, caractérisé en ce que les émulsifiants sont des agents tensio-actifs non ionogènes et, en particulier, ceux choisis dans le groupe formé de :
(B1) Les produits d'addition de 2 à 30 moles d'oxyde d'éthylène et/ou de 0 à 5 moles d'oxyde de propylène avec des alcools gras linéaires ayant de 8 à 22 atomes de carbone, des acides gras ayant de 12 à 22 atomes de carbone et des alkylphénols ayant de 8 à 15 atomes de carbone dans le groupe alkyle.
(B2) Les mono- et diesters d'acides gras en $C_{12}$-$C_{22}$ desproduits d'addition de 1 à 30 moles d'oxyde d'éthylène avec la glycérine.
(B3) Les mono- et diesters de glycérine et les mono- et diesters de sorbitane des acides gras saturés et non saturés en $C_8$-$C_{22}$ et leurs produits d'addition avec l'oxyde d'éthylène.
(B4) Les mono- et oligoglycosides d'alkyle en $C_8$-$C_{22}$ et leurs analogues éthoxylés; et
(B5) Les produits d'addition de 5 à 60 moles d'oxyde d'éthylène avec l'huile de ricin et l'huile de ricin durcie.

**7.** Concentré à éclat nacré selon l'une des revendications 1 à 5, caractérisé en ce que les émulsifiants sont des agents tensio-actifs zwitterioniques et, en particulier, ceux choisis dans le groupes des bétaïnes.

**8.** Concentré a éclat nacré selon l'une des revendications 1 à 4, caractérisé en ce que les émulsifiants contenus sont des émulsifiants anioniques.

**9.** Concentré à éclat nacré selon l'une des revendications 1 à 8, caractérisé en ce que l'alcool est choisi dans le groupe constitué de la glycérine, du 1,2-propylèneglycol, du 1,3-propylèneglycol et de la sorbite.

**10.** Concentré à éclat nacré selon l'une des revendications 1 à 9, caractérisé en ce que l'alcool est la glycérine.

**11.** Procédé de préparation de concentrés à éclat nacré selon l'une des revendications 1 à 10, caractérisé en ce qu'on chauffe un mélange des composants (A), (B) et (C) à une température située de 1 à 30 °C au-dessus du point de fusion du mélange, qu'on y ajoute la quantité d'eau nécessaire, à environ la même température et qu'on refroidit ensuite à température ambiante.

**12.** Procédé de fabrication de préparations aqueuses liquides, opales et à éclat nacré de sustances tensio-actives solubles dans l'eau, caractérisé en ce que l'on ajoute aux préparations aqueuses transparentes, à une température de 0 à 40 °C, des concentrés à éclat nacré selon l'une des revendications 1 à 10 en quantités de 0,5 à 10% en poids et, en particulier, de 1,5 à 5% en poids et qu'on les répartit par agitation.